# EUROPEAN PATENT APPLICATION

(11) **EP 3 771 432 A1**
(43) Date of publication of application: **03.02.2021**
(21) Application number: 19189282.7
(22) Date of filing: 31.07.2019
(51) Int. Cl.: A61B 8/00, A61B 8/08

(54) **RELATIVE LOCATION DETERMINING FOR PASSIVE ULTRASOUND SENSORS**

(71) Applicant: Koninklijke Philips N.V., 5656 AG Eindhoven (NL)
(72) Inventor: BHARAT, Shyam, 5656 AE Eindhoven (NL); VAIDYA, Kunal, 5656 AE Eindhoven (NL); ERKAMP, Ramon Quido, 5656 AE Eindhoven (NL); JAIN, Ameet Kumar, 5656 AE Eindhoven (NL)
(74) Representative: de Haan, Poul Erik

(57) **Abstract**

A controller (250) for identifying out-of-plane motion of a passive ultrasound sensor (S1) relative to an imaging plane from an ultrasound imaging probe includes a memory (391) that stores instructions and a processor (392) that executes the instructions. When executed by the processor, the instructions cause a system that includes the controller (250) to implement a process that includes obtaining (S710), from a position and orientation sensor (212) fixed to the ultrasound imaging probe (210), measurements of motion of the ultrasound imaging probe (210) between a first point in time and a second point in time. The process implemented by the controller (250) also includes obtaining (S720) intensity of signals received by the passive ultrasound sensor (S1) at the first point in time and at the second point in time based on emissions of beams from the ultrasound imaging probe (210), and determining (S730), based on the measurements of motion and the intensity of signals, directionality of and distance from the passive ultrasound sensor (S1) to the imaging plane.

## Description

### BACKGROUND OF THE INVENTION

Ultrasound tracking technology estimates the position of a passive ultrasound sensor (e.g., PZT, PVDF, copolymer or other piezoelectric material) in the field of view (FOV) of a diagnostic ultrasound B-mode image by analyzing the signal received by the passive ultrasound sensor as imaging beams from an ultrasound probe sweep the field of view. A passive ultrasound sensor is an acoustic pressure sensor, and these passive ultrasound sensors are used to determine location of an interventional medical device. Time-of-flight measurements provide the axial/radial distance of the passive ultrasound sensor from an imaging array of the ultrasound probe, while amplitude measurements and knowledge of the direct beam firing sequence provide the lateral / angular position of the passive ultrasound sensor.

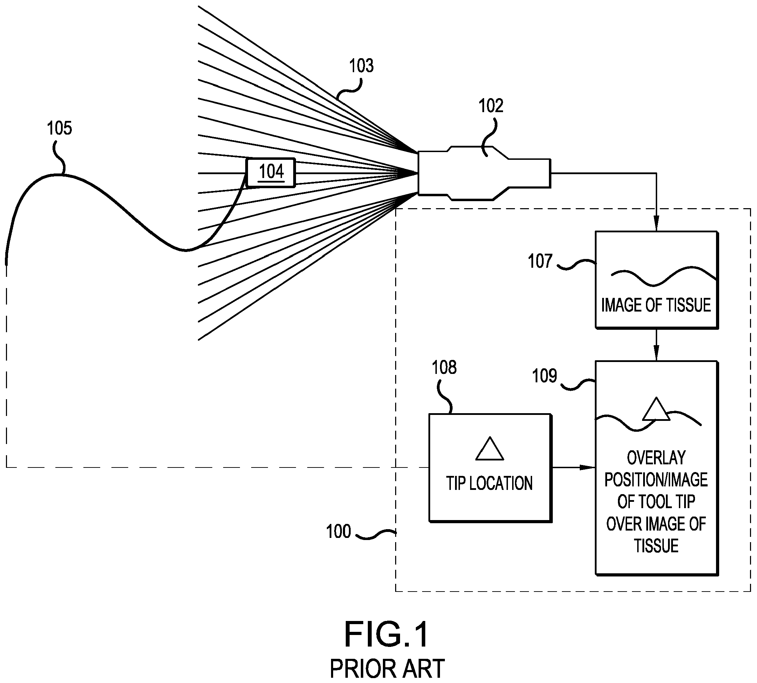

FIG. 1 illustrates a known system for tracking an interventional medical device using a passive ultrasound sensor. The known system in FIG. 1 may be known by the name "Insitu", which stands for Intelligent Sensing of Tracked Instruments using Ultrasound. In FIG. 1, an ultrasound probe 102 emits an imaging beam 103 that sweeps across a passive ultrasound sensor 104 on a tip of an interventional medical device 105. An image of tissue 107 is fed back by the ultrasound probe 102. A location of the passive ultrasound sensor 104 on the tip of the interventional medical device 105 is provided as a tip location 108 upon determination by a signal processing algorithm. The tip location 108 is overlaid on the image of tissue 107 as an overlay image 109. The image of tissue 107, the tip location 108, and the overlay image 109 are all displayed on a display 100.

Currently, the response of the passive ultrasound sensor 104 is symmetric around the ultrasound (US) imaging plane, thus making it impossible to determine which side of the imaging plane the interventional medical device 105 is on. That is, a voltage reading from the passive ultrasound sensor 104 may be identical whether it is on a first side of an ultrasound imaging plane or a second side of the ultrasound imaging plane opposite the first side. In isolation, the voltage reading as a response of the passive ultrasound sensor 104 does not provide sufficient information. Moreover, the known system in FIG. 1 does not provide a quantitative indication of the out-of-plane (OOP) distance from the imaging plane of/from the ultrasound probe 102 to the passive ultrasound sensor 104. The out-of-plane distance may be important for certain applications.

The known system in FIG. 1 is also unable to differentiate translational motion (movement between two points in space) from rotational motion (movement about an axis) based on the signal voltage alone, which leads to confusion. Out-of-plane rotation and translation may instead be estimated by measurements via a number of other existing methods in a relative (non-absolute) manner. For example, electromagnetic or optical tracking sensors can be attached to the ultrasound probe and provide accurate measurements of probe absolute translation and rotation. Similarly, inertial motion unit (IMU) sensors may be integrated into an ultrasound probe to determine the pose of the ultrasound probe. Unlike electromagnetic or optical tracking sensors, inertial motion unit sensor measurements are relative; that is, rather than measuring the absolute probe pose, inertial motion unit measurements provide the relative frame-to-frame change in the pose, which are then used to indirectly estimate the absolute pose. A drawback of this approach is that the pose estimates can be inaccurate due to drift and incremental build-up of error. This is primarily due to the need for double-integration of the acceleration measurements to derive position insofar as minute errors in the acceleration lead to the accumulation of error in the position determination over time. Yet another method to measure probe translation and rotation is to make use of features in the ultrasound image itself. In-plane motion can typically be computed from the image information in a straightforward manner by tracking image intensities directly. Out-of-plane motion is typically estimated by observing the decorrelation of acoustic speckle features between image frames, with increased decorrelation corresponding to increased out-of-plane motion.

A significant body of literature has focused on methods to combine sensor (electromagnetic, optical, and/or IMU) based tracking and image-based methods to estimate ultrasound transducer pose and therefore enable three-dimensional volume reconstruction. A fundamental technical challenge with existing approaches, particularly if the tracking sensor provides relative estimates rather than absolute measurements (as in the case of IMU tracking), is the lack of a reliable reference marker within the three-dimensional volume of interest. Without such a marker, relative estimates based on a sensor and image-based estimates are both prone to error and uncertainty; as such, the result of their combination is likewise uncertain and error-prone. Thus, the reference marker within the ultrasound volume can serve as a constraint on the volume reconstruction process and improve the accuracy of the volume reconstruction.

Inversely, when the reference marker within the ultrasound volume is relative rather than absolute (as is in the case of the known system in FIG 1), the estimation of ultrasound probe motion and ultrasound volume reconstruction can provide quantitative indication of the out-of-plane (OOP) distance from the imaging plane of/from the ultrasound probe 102 to the passive ultrasound sensor 104. Similarly, the ultrasound probe motion and volume reconstruction can be used to determine which side of the imaging plane that the interventional medical device 105 is on.

### SUMMARY OF THE INVENTION

According to an aspect of the present disclosure, a controller for identifying out-of-plane motion of a passive ultrasound sensor relative to an imaging plane from an ultrasound imaging probe includes a memory and a processor. The memory stores instructions. The processor executes the instructions. When executed by the processor, the instructions cause a system that includes the controller to implement a process that includes obtaining, from a position and orientation sensor fixed to the ultrasound imaging probe, measurements of motion of the ultrasound imaging probe between a first point in time and a second point in time. The process implemented when the processor executes the instructions also includes obtaining intensity of signals received by the passive ultrasound sensor at the first point in time and at the second point in time based on emissions of beams from the ultrasound imaging probe. The process implemented when the processor executes the instructions further includes determining, based on the measurements of motion and the intensity of signals, directionality of and distance from the passive ultrasound sensor to the imaging plane.

According to another aspect of the present disclosure, a tangible non-transitory computer readable storage medium stores a computer program. When executed by a processor, the computer program causes a system that includes the tangible non-transitory computer readable storage medium to perform a process for identifying out-of-plane motion of a passive ultrasound sensor relative to an imaging plane from an ultrasound imaging probe. The process performed when the processor executes the computer program from the tangible non-transitory computer readable storage medium includes obtaining, from a position and orientation sensor fixed to the ultrasound imaging probe, measurements of motion of the ultrasound imaging probe between a first point in time and a second point in time. The process performed when the processor executes the computer program from the tangible non-transitory computer readable storage medium also includes obtaining intensity of signals received by the passive ultrasound sensor at the first point in time and at the second point in time based on emissions of beams from the ultrasound imaging probe. The process performed when the processor executes the computer program from the tangible non-transitory computer readable storage medium further includes determining, based on the measurements of motion and the intensity of signals, directionality of and distance from the passive ultrasound sensor to the imaging plane.

According to another aspect of the present disclosure, a system for identifying out-of-plane motion of a passive ultrasound sensor relative to an imaging plane from an ultrasound imaging probe includes an ultrasound imaging probe, a position and orientation sensor, a passive ultrasound sensor, and a controller. The ultrasound imaging probe emits beams during a medical intervention. The position and orientation sensor is fixed to the ultrasound imaging probe. The passive ultrasound sensor is fixed to an interventional medical device during the medical intervention. The controller includes a memory that stores instructions and a processor that executes the instructions. When executed by the processor, the instructions cause the system to implement a process that includes obtaining, from the position and orientation sensor, measurements of motion of the ultrasound imaging probe between a first point in time and a second point in time. The process implemented when the processor executes the instructions also includes obtaining intensity of signals received by the passive ultrasound sensor at the first point in time and at the second point in time based on emissions of beams from the ultrasound imaging probe. The process implemented when the processor executes the instructions further includes determining, based on the measurements of motion and the intensity of signals, directionality of and distance from the passive ultrasound sensor to the imaging plane.

### BRIEF DESCRIPTION OF THE DRAWINGS

The example embodiments are best understood from the following detailed description when read with the accompanying drawing figures. It is emphasized that the various features are not necessarily drawn to scale. In fact, the dimensions may be arbitrarily increased or decreased for clarity of discussion. Wherever applicable and practical, like reference numerals refer to like elements.
FIG. 1 illustrates a known system for tracking an interventional medical device using a passive ultrasound sensor.
FIG. 2A illustrates a system for relative location determining for passive ultrasound sensors, in accordance with a representative embodiment.
FIG. 2B illustrates a process for relative location determining for passive ultrasound sensors, in accordance with a representative embodiment.
FIG. 3 illustrates another system for relative location determining for passive ultrasound sensors, in accordance with a representative embodiment, in accordance with a representative embodiment.
FIG. 4 illustrates geometric configurations with varying outcomes for relative location determining for passive ultrasound sensors, in accordance with a representative embodiment.
FIG. 5 illustrates another process for relative location determining for passive ultrasound sensors, in accordance with a representative embodiment.
FIG. 6A illustrates input data for obtaining a three-dimensional probe pose in relative location determining for passive ultrasound sensors, in accordance with a representative embodiment.
FIG. 6B illustrates inputs and outputs for joint optimization for obtaining a three-dimensional probe pose in relative location determining for passive ultrasound sensors, in accordance with a representative embodiment.
FIG. 7 illustrates another process for relative location determining for passive ultrasound sensors, in accordance with a representative embodiment.
FIG. 8 illustrates another process for relative location determining for passive ultrasound sensors, in accordance with a representative embodiment.
FIG. 9 illustrates another process for relative location determining for passive ultrasound sensors, in accordance with a representative embodiment.
FIG. 10 illustrates another process for relative location determining for passive ultrasound sensors, in accordance with a representative embodiment.

### DETAILED DESCRIPTION OF THE EMBODIMENTS

In the following detailed description, for purposes of explanation and not limitation, representative embodiments disclosing specific details are set forth in order to provide a thorough understanding of an embodiment according to the present teachings. Descriptions of known systems, devices, materials, methods of operation and methods of manufacture may be omitted so as to avoid obscuring the description of the representative embodiments. Nonetheless, systems, devices, materials and methods that are within the purview of one of ordinary skill in the art are within the scope of the present teachings and may be used in accordance with the representative embodiments. It is to be understood that the terminology used herein is for purposes of describing particular embodiments only and is not intended to be limiting. The defined terms are in addition to the technical and scientific meanings of the defined terms as commonly understood and accepted in the technical field of the present teachings.

It will be understood that, although the terms first, second, third etc. may be used herein to describe various elements or components, these elements or components should not be limited by these terms. These terms are only used to distinguish one element or component from another element or component. Thus, a first element or component discussed below could be termed a second element or component without departing from the teachings of the inventive concept.

The terminology used herein is for purposes of describing particular embodiments only and is not intended to be limiting. As used in the specification and appended claims, the singular forms of terms 'a', 'an' and 'the' are intended to include both singular and plural forms, unless the context clearly dictates otherwise. Additionally, the terms "comprises", and/or "comprising," and/or similar terms when used in this specification, specify the presence of stated features, elements, and/or components, but do not preclude the presence or addition of one or more other features, elements, components, and/or groups thereof. As used herein, the term "and/or" includes any and all combinations of one or more of the associated listed items.

Unless otherwise noted, when an element or component is said to be "connected to", "coupled to", or "adjacent to" another element or component, it will be understood that the element or component can be directly connected or coupled to the other element or component, or intervening elements or components may be present. That is, these and similar terms encompass cases where one or more intermediate elements or components may be employed to connect two elements or components. However, when an element or component is said to be "directly connected" to another element or component, this encompasses only cases where the two elements or components are connected to each other without any intermediate or intervening elements or components.

In view of the foregoing, the present disclosure, through one or more of its various aspects, embodiments and/or specific features or sub-components, is thus intended to bring out one or more of the advantages as specifically noted below. For purposes of explanation and not limitation, example embodiments disclosing specific details are set forth in order to provide a thorough understanding of an embodiment according to the present teachings. However, other embodiments consistent with the present disclosure that depart from specific details disclosed herein remain within the scope of the appended claims. Moreover, descriptions of well-known apparatuses and methods may be omitted so as to not obscure the description of the example embodiments. Such methods and apparatuses are within the scope of the present disclosure.

As described herein, combining voltage measurements of passive ultrasound sensors with sensor and/or image-based measurements may allow quantitative measurements of out-of-plane distance and directionality in a reliable manner. The position information corresponding to a location of an interventional medical device may be from the position of a passive ultrasound sensor or derived in alternative ways such as by electromagnetic measurements or image analysis. The position is used as a high accuracy reference marker that constrains the sensor-and/or image-based measurements around the position with the assumption that the position remains stationary. In an extension of the teachings herein, a three-dimensional volume may be reconstructed around the position.

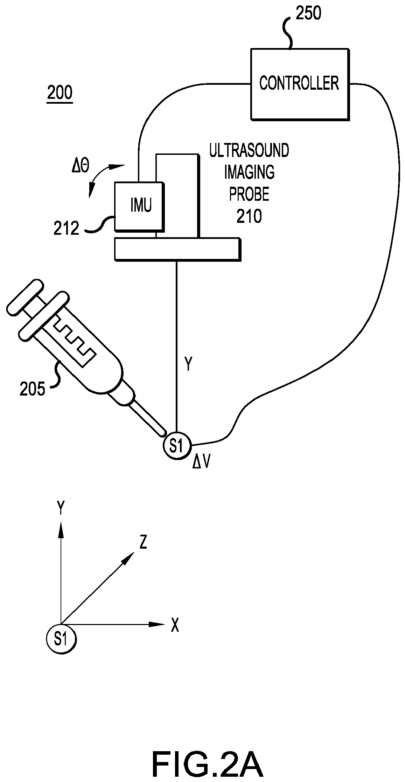

FIG. 2A illustrates a system for relative location determining for passive ultrasound sensors, in accordance with a representative embodiment.

In FIG. 2A, a system 200 includes an interventional medical device 205, an ultrasound imaging probe 210, an inertial motion unit 212 (an IMU sensor), a controller 250, and a passive ultrasound sensor S1. While most embodiments herein describe the use of a passive ultrasound sensor S1 positioned in a stationary manner within the field of view of the ultrasound imaging probe 210 and an inertial motion unit 212 sensor fixed to the ultrasound imaging probe 210, other types of sensors or equipment may be used to identify, for example, location of the tip of the interventional medical device 205 or three-dimensional motion of the ultrasound imaging probe 210. For example, electromagnetic sensors or image analysis techniques may be used to identify the location of the tip of the interventional medical device 205 without departing from the scope and spirit of the present disclosure.

The interventional medical device 205 may be a needle but is representative of numerous different types of interventional medical devices that can be inserted into a subject during a medical intervention. The passive ultrasound sensor S1 is attached to or incorporated within the interventional medical device 205.

The ultrasound imaging probe 210 may include a beamformer used to generate and send an ultrasound beam via an imaging array of transducers. Alternatively, the ultrasound imaging probe 210 may receive beamformer data from, e.g., a console, and use the beamformer data to generate and send the ultrasound beam via the imaging array of transducers. The ultrasound beam emitted from the ultrasound imaging probe 210 includes an imaging plane that is or may be aligned with and centered along the primary axis of the ultrasound imaging probe 210. In FIG. 2A, the primary axis of the ultrasound imaging probe 210 and thus the imaging plane is shown as the vertical direction labelled Y. The ultrasound imaging probe 210 receives or may also receive reflections of the imaging beam that are reflected from the subject of the interventional procedure. As is known, the received reflections of the imaging beam are used to generate ultrasound images of the subject of the interventional procedure.

The inertial motion unit 212 is attached to or incorporated within the ultrasound imaging probe 210. The inertial motion unit 212 may include a gyroscope and an accelerometer and is or may be mounted to the ultrasound imaging probe 210 to assist in estimating the pose of the ultrasound imaging probe 210. An accelerometer measures three-dimensional translations of the ultrasound imaging probe 210. A gyroscope measures three-dimensional rotations of the ultrasound imaging probe 210. The inertial motion unit 212 may detect, determine, calculate or otherwise identify movement of the ultrasound imaging probe 210 in three-dimensional translational coordinates such as horizontal, vertical and depth. The inertial motion unit may also detect, determine, calculate or otherwise identify movement of the ultrasound imaging probe 210 in three rotational components (Euler angles). As described herein, an inertial motion unit 212 and an inertial motion unit 312 are both examples of position and orientation sensors which can be used to identify position and/or orientation of the interventional medical device 205. Such position and orientation sensors include instantiations that are not necessarily attached to or contained within an interventional medical device 205, and may include cameras and image processing equipment that can determine position and/or orientation of the interventional medical device 205, for example.

The controller 250 may be an electronic device with a memory that stores instructions and a processor that executes the instructions to implement some or all aspects of processes described herein. The controller 250 receives or may receive measurements (e.g., voltage readings) from the passive ultrasound sensor S1 and motion readings of the translational and rotational movement from the inertial motion unit 212. The controller 250 identifies out-of-plane directionality of and distance from the passive ultrasound sensor S1 relative to an imaging plane from the ultrasound imaging probe 210 using the received measurements and motion readings. The identification of out-of-plane directionality and out-of-plane distance are explained below in detail, along with three-dimensional volume reconstruction based on the received measurements and/or motion readings and other practical applications made possible with the teachings herein.

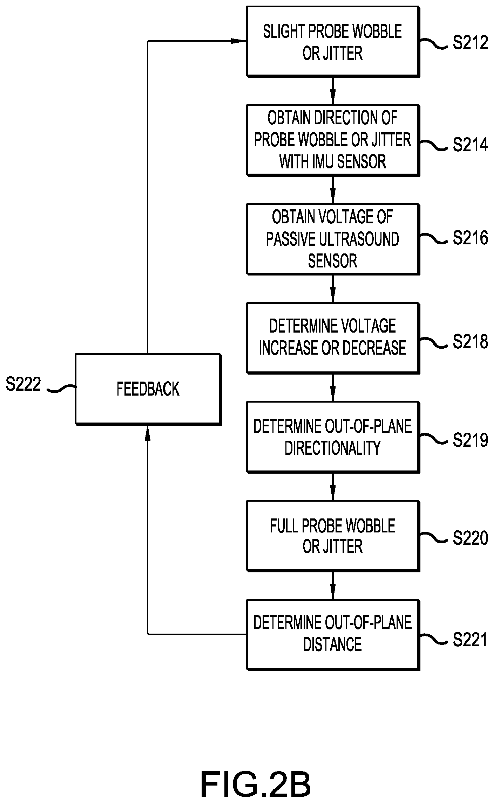

While FIG. 2A illustrates a system for relative location determining for passive ultrasound sensors, FIG. 2B illustrates a process for relative location determining for passive ultrasound sensors, in accordance with a representative embodiment.

More particularly, FIG. 2B illustrates an overview process showing how measurements (e.g. of voltage) from a passive ultrasound sensor S1 and motion readings from an inertial motion unit 212 can be used to determine out-of-plane directionality and out-of-plane distance for the passive ultrasound sensor S1. Other methods of probe tracking, such as electromagnetic tracking, optical tracking, and image or speckle analysis, may be used in place of the inertial motion unit 212. Other methods may be implemented in part using a controller 250, but also may involve additional equipment not shown in FIG. 2. For example, image analysis may be performed by a dedicated processor other than a processor in the controller 250, such that the results of the image analysis are provided to the controller 250 as information informing of the results of tracking both translational and rotational movement by the ultrasound imaging probe 210.

In the process of FIG. 2B, the process starts at S212 with a slight probe wobble or jitter of the ultrasound imaging probe 210. That is, a user intentionally or unintentionally wobbles or jitters the ultrasound imaging probe 210 at least slightly at S212, and the inertial motion unit 212 (or other probe tracking sensor or image/speckle analysis mechanism) generates readings, for instance of the accelerometer and the gyroscope, that change based on the wobble or jitter.

The process of FIG. 2B next moves to S214 to obtain a directionality of the probe wobble or jitter with the inertial motion unit 212. That is, at S214 readings of the inertial motion unit 212 are obtained throughout the duration of the wobble, including for times corresponding to the beginning and end of the wobble at S212, and the readings of the inertial motion unit 212 reflect a directionality of the probe wobble or jitter. The directionality obtained at S214 may be obtained by functionality of the inertial motion unit 212, and particularly by functionality used to obtain translational movement in 3 translational dimensions and rotational movement in 3 rotational dimensions. Alternatively, the directionality obtained at S214 may be obtained by the controller 250 based on readings of the translational movement and the rotational movement by the inertial motion unit 212.

At S216, the process of FIG. 2B obtains a voltage of the passive ultrasound sensor S1. In reality, the voltage of the passive ultrasound sensor S1 may be read for each imaging beam that reaches the passive ultrasound sensor S1, but for the purposes of the description for FIG. 2B an individual voltage reading is obtained. As explained below, the individual voltage reading is compared with a previous individual voltage reading, such as the reading of the individual voltage immediately previous. The position of the passive ultrasound sensor S1 can be determined from the voltage obtained at S216 and used as an accurate reference marker for estimating the pose of the ultrasound imaging probe 210 based on readings of the inertial motion unit 212 at S214. That is, determinations as to whether voltage received by a passive ultrasound sensor S1 increase or decrease can be used for determining the out-of-plane directionality of the passive ultrasound sensor S1, as explained below.

Next, at S218 the process of FIG. 2B determines whether the voltage obtained at S216 increased or decreased compared to the previous voltage reading. The determination at S218 may be by the controller 250 and may involve simple iterative comparisons of each voltage reading with the previous voltage reading.

For simple out-of-plane directionality estimation, an algorithm can be used at S219 to compare the out-of-plane motion estimated by the inertial motion unit 212 at S214 with the signal intensity of the response of the passive ultrasound sensor S1 at S216. For out-of-plane directionality from the passive ultrasound sensor S1 relative to the ultrasound imaging plane, if the voltage increases with a rotation away from the arbitrary sensor axis, the passive ultrasound sensor S1 is on the same side as the rotation. If the voltage decreases with a rotation away from the arbitrary sensor axis, the passive ultrasound sensor S1 is on the opposite side as the rotation. If the voltage increases with a rotation toward the arbitrary sensor axis, the passive ultrasound sensor S1 is on the same side as the rotation. If the voltage decreases with a rotation toward the arbitrary sensor axis, the passive ultrasound sensor S1 is on the opposite side as the rotation.

At S220, the process of FIG. 2B includes a full probe wobble or jitter. The full probe wobble or jitter at S220 is used to obtain the out-of-plane distance separate from the out-of-plane directionality obtained earlier. Here, the user may be instructed to wobble/rotate the ultrasound imaging probe 210 without sliding/translating too much across the skin.

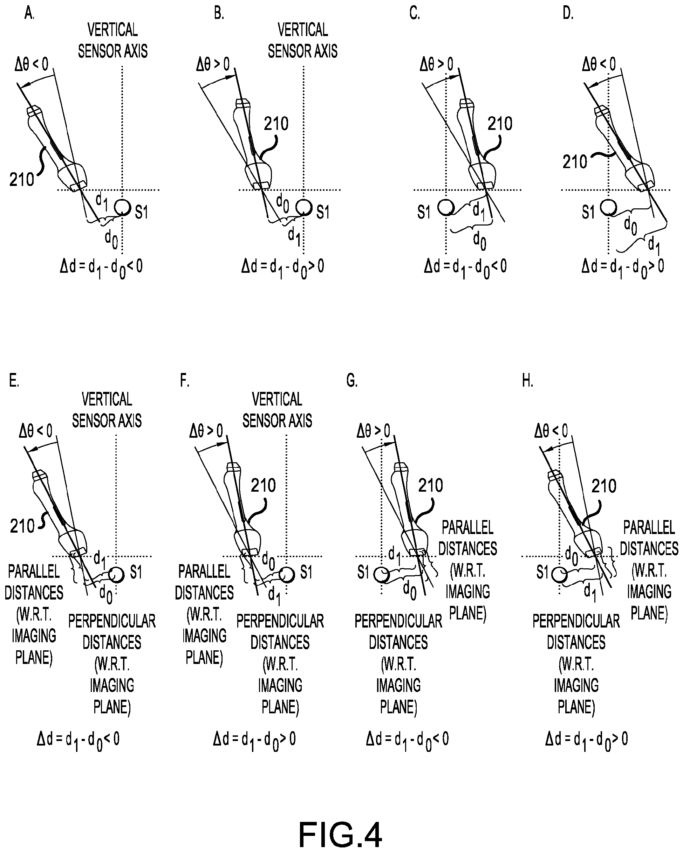

The out-of-plane distance is determined at S221. The process for obtaining the full out-of-plane distance at S221 is analogous to, but not the same as, calculating the length of a leg of a triangle. At S221, the absolute out-of-plane distance can be approximated by assuming that the axis of out-of-plane rotation is aligned with the head of the ultrasound imaging probe (i.e. the transducer element array is in direct contact with the skin). The out-of-plane distance is estimated based on the rotational component of the inertial motion unit pose output. Here, since the depth of the passive ultrasound sensor S1 in the ultrasound image is known via the location system for the passive ultrasound sensor S1, and since the rotational component is known from the angle of rotation, the out-of-plane distance can be computed accordingly. The out-of-plane accuracy may be within 1 millimeter (mm) or less, assuming no sliding/translation motions during the wobble. Examples of the geometry used to calculate the absolute out-of-plane distance at S221 are shown in and explained with respect to FIG. 4.

The out-of-plane distance determination at S221 may be a calculation as part of a process executed by a controller. The process may include calculating a change in distance of a passive ultrasound sensor S1 from the imaging plane. The change in distance may be calculated based on rotation of the position and orientation sensor (e.g., IMU) relative to the fixed axis of the passive ultrasound sensor S1 and the distance between the passive ultrasound sensor S1 and the ultrasound imaging probe 210. The distance of the passive ultrasound sensor S1 from the imaging plane may be determined from a fixed point on the fixed axis through the passive ultrasound sensor S1 to an intersection between the imaging plane and a line perpendicular to the fixed axis from the fixed point.

In an embodiment, the determination of out-of-plane distance at S221 is performed based on the same wobble at S212 as is used for the determination of out-of-plane directionality at S219. In other words, the full wobble at S220 may be unnecessary when enough information is determined or determinable from the wobble at S212.

Feedback is obtained at S222 in the process of FIG. 2B, and then the process returns to S212 with another slight probe wobble or jitter. Feedback may include visualizations provided on a monitor, including differentiated visualizations that vary based on the results of the processes described herein. Given the iterative and recurring process shown in FIG. 2B, the calculation of out-of-plane directionality and out-of-plane distance for a passive ultrasound sensor S1 may be performed repeatedly during an interventional procedure.

As described above, for the process of FIG. 2B the user wobbles the probe slightly at S212, creating out-of-plane motion. Alternatively, the natural freehand motion/jitter may also be used as the basis at S212 if sensitivity of the inertial motion unit 212 is sufficient. The measurements of the inertial motion unit 212 captured at S214 are used to determine the three translational components and three rotational components (Euler angles) of the ultrasound imaging probe 210. The translational and rotational components that correspond to the motion of the ultrasound imaging probe 210 is/are extracted at S214 to determine the angle of rotation. For each measurement of the inertial motion unit 212, the corresponding voltage of the passive ultrasound sensor S1 is also recorded at S216. The change in the out-of-plane rotational angle of the inertial motion unit 212 obtained at S214 is compared to the corresponding change in the voltage of the passive ultrasound sensor from S216, and the relative information from S214 and S218 is used at S219 and S221 to determine out-of-plane directionality and distance for the passive ultrasound sensor S1.

The process of FIG. 2B may be run in a fully automatic and continuous manner without any input needed from the user. As a result of the determinations at S219, the system 200 may generate different visualizations for the passive ultrasound sensor S1 to be shown on an electronic display. A displayed representation of the passive ultrasound sensor S1 may be controlled by a controller to vary based on which side of an imaging plane the passive ultrasound sensor S1 is on. For example, the user may be shown a different colored circle depending on which side of the imaging plane the passive ultrasound sensor S1 is found. Crossing of the imaging plane may even be determined by the controller 250 by tracking when the voltage readings of the passive ultrasound sensor S1 reach a maximum before decreasing again.

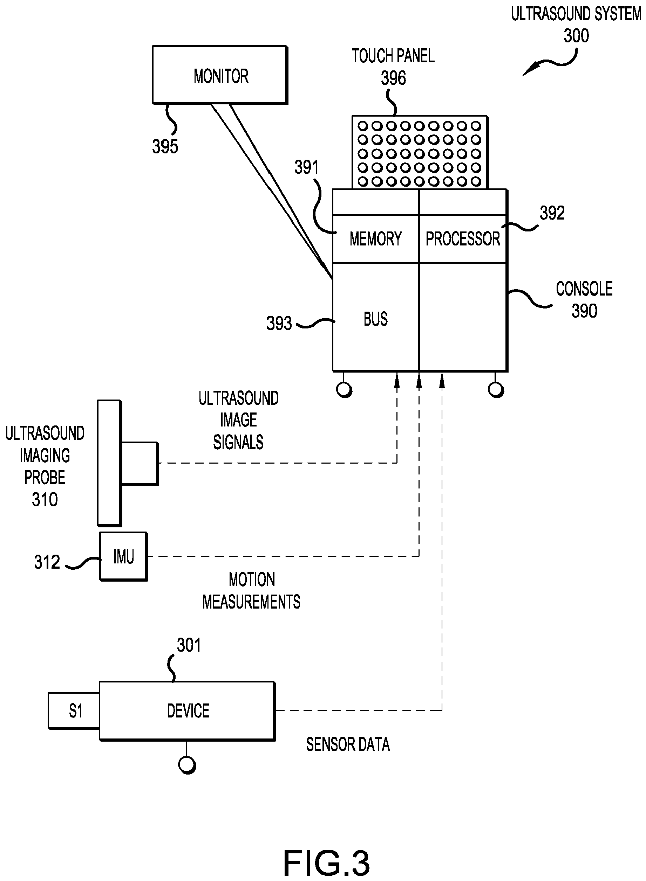

FIG. 3 illustrates another system for relative location determining for passive ultrasound sensors, in accordance with a representative embodiment, in accordance with a representative embodiment.

The system 300 in FIG. 3 includes an ultrasound imaging probe 310, an inertial motion unit 312, an interventional medical device 301, a passive ultrasound sensor S1, a console 390 and a monitor 395. The console 390 includes a memory 391 that stores instructions, a processor 392 that executes the instructions, a bus 393 for carrying data and instructions within the console 390, and a touch panel 396 for a user to input data and instructions and for the console 390 to output data and instructions. The combination of the memory 391 and the processor 392 may be part of a controller such as the controller 250 in the embodiment of FIG. 2. However, a controller such as the controller 250 in the embodiment of FIG. 2 does not have to be implemented in a console 390, and instead may be implemented in a separate computer such as a PC or laptop, or in another type of device. Regardless of how or where a controller 250 is implemented, a controller 250 may be implemented with a combination of memory 391 and a processor 392 as described herein

In the system 300 of FIG. 3, the passive ultrasound sensor S1 provides sensor data to the console 390, and specifically for analysis by the processor 392 in accordance with the instructions in the memory 391. The inertial motion unit 312 (or other probe tracking sensor or image/speckle analysis mechanism) provides motion measurements to the console 390, and specifically for analysis by the processor 392 in accordance with the instructions in the memory 391. Of course, when other mechanisms are used to track the ultrasound imaging probe 310, the other mechanisms may include processing outside of the console 390 or at least separate from the memory 391 and the processor 392. For example, image processing used to track the ultrasound imaging probe 310 may be performed by a dedicated image analysis processor that provides rotational and translational movement of the ultrasound imaging probe 310 to the processor 392 for processing in accordance with the descriptions herein. The ultrasound imaging probe 310 operates in accordance with known capabilities of ultrasound imaging probes so as to send ultrasound image signals to the console 390 to display ultrasound images to a user such as a medical professional or the subject of the interventional procedure.

In the embodiment of FIG. 3, beamforming may be performed by the console 390, and beamforming instructions such as a sequence and pattern for a series of beams may be sent from the console 390 to the ultrasound imaging probe 310. Additionally, ultrasound imagery may be sent from the ultrasound imaging probe 310 to the console 390. The passive ultrasound sensor S1 may provide voltage readings for each beam to the console 390. The inertial motion unit 312 may provide motion readings to the console 390. Accordingly, a controller in the console 390 or otherwise may implement part or all of the processes described herein. For example, a controller in the console 390 may determine the out-of-plane directionality at S219 and determine the out-of-plane distance at S221, both as described with respect to FIG. 2B.

A processor 392 for a controller is tangible and non-transitory. As used herein, the term "non-transitory" is to be interpreted not as an eternal characteristic of a state, but as a characteristic of a state that will last for a period. The term "non-transitory" specifically disavows fleeting characteristics such as characteristics of a carrier wave or signal or other forms that exist only transitorily in any place at any time. A processor is an article of manufacture and/or a machine component. A processor 392 for a controller is configured to execute software instructions to perform functions as described in the various embodiments herein. A processor 392 for a controller may be a general-purpose processor or may be part of an application specific integrated circuit (ASIC). A processor 392 for a controller may also be a microprocessor, a microcomputer, a processor chip, a controller, a microcontroller, a digital signal processor (DSP), a state machine, or a programmable logic device. A processor 392 for a controller may also be a logical circuit, including a programmable gate array (PGA) such as a field programmable gate array (FPGA), or another type of circuit that includes discrete gate and/or transistor logic. A processor 392 for a controller may be a central processing unit (CPU), a graphics processing unit (GPU), or both. Additionally, any processor described herein may include multiple processors, parallel processors, or both. Multiple processors may be included in, or coupled to, a single device or multiple devices. A "processor" as used herein encompasses an electronic component which is able to execute a program or machine executable instruction. References to the computing device comprising "a processor" should be interpreted as possibly containing more than one processor or processing core. The processor may for instance be a multi-core processor. A processor may also refer to a collection of processors within a single computer system or distributed amongst multiple computer systems. The term computing device should also be interpreted to possibly refer to a collection or network of computing devices each including a processor or processors. Many programs have instructions performed by multiple processors that may be within the same computing device or which may even be distributed across multiple computing devices.

Memories described herein are tangible storage mediums that can store data and executable instructions and are non-transitory during the time instructions are stored therein. As used herein, the term "non-transitory" is to be interpreted not as an eternal characteristic of a state, but as a characteristic of a state that will last for a period. The term "non-transitory" specifically disavows fleeting characteristics such as characteristics of a carrier wave or signal or other forms that exist only transitorily in any place at any time. A memory described herein is an article of manufacture and/or machine component. Memories described herein are computer-readable mediums (computer-readable storage mediums) from which data and executable instructions can be read by a computer. Memories as described herein may be random access memory (RAM), read only memory (ROM), flash memory, electrically programmable read only memory (EPROM), electrically erasable programmable read-only memory (EEPROM), registers, a hard disk, a removable disk, tape, compact disk read only memory (CD-ROM), digital versatile disk (DVD), floppy disk, blu-ray disk, or any other form of storage medium known in the art. Memories may be volatile or non-volatile, secure and/or encrypted, unsecure and/or unencrypted. "Memory" is an example of a computer-readable storage medium. Computer memory is any memory which is directly accessible to a processor. Examples of computer memory include, but are not limited to RAM memory, registers, and register files. References to "computer memory" or "memory" should be interpreted as possibly being multiple memories. The memory may for instance be multiple memories within the same computer system. The memory may also be multiple memories distributed amongst multiple computer systems or computing devices.

FIG. 4 illustrates geometric configurations with varying outcomes for relative location determining for passive ultrasound sensors, in accordance with a representative embodiment.

The geometric configurations in FIG. 4 are broken up into four possible outcomes in eight separate visualizations A, B, C, D, E, F, G and H. The four possible outcomes are shown in visualizations A, B, C and D in FIG. 4, and the outcome of visualization A, B, C, and D in FIG. 4 are detailed alternatively in visualizations E, F, G, and H. The possible outcomes vary based on the output from the passive ultrasound sensor S1 and the inertial motion unit 212, which in turn reflect the directionality of out-of-plane translational movement indicated by the data (e.g., voltage readings) from the passive ultrasound sensor S1 and the directionality of out-of-plane rotational movement from the data (e.g., gyroscope readings) from the inertial motion unit 212. In each of visualizations A, B, C, D, E, F, G and H in FIG. 4, the passive ultrasound sensor S1 is indicated as a circle, a thin line reflects the arbitrary, fixed axis of the passive ultrasound sensor S1 (e.g., a vertical axis), and the ultrasound imaging probe 210 is indicated by an outline. As explained previously, a primary axis through an ultrasound imaging probe 210 may correspond to the imaging plane from the ultrasound imaging probe 210. In the first outcome, if the voltage detected by the passive ultrasound sensor S1 increases with a rotation detected by the inertial motion unit 212 away from the arbitrary, fixed sensor axis, the passive ultrasound sensor S1 is on the same side as the rotation. In the second outcome, if the voltage decreases with a rotation away from the arbitrary, fixed sensor axis, the passive ultrasound sensor S1 is on the opposite side as the rotation. In a third outcome, if the voltage increases with a rotation toward the arbitrary, fixed sensor axis, the passive ultrasound sensor S1 is on the same side as the rotation. In a fourth outcome, if the voltage decreases with a rotation toward the arbitrary, fixed sensor axis, the passive ultrasound sensor S1 is on the opposite side as the rotation.

In the visualizations A, B, C and D of FIG. 4, the rotation of the ultrasound imaging probe 210 is shown as ΔΘ. A first distance do is the horizontal distance from a fixed point on the passive ultrasound sensor S1 to the first imaging plane from the ultrasound imaging probe 210 at the first time. A second distance d₁ is the horizontal distance from the fixed point on the passive ultrasound sensor S1 to the second imaging plane from the ultrasound imaging probe 210 at the second time. The change from the first distance do and the second distance d₁ determines the directionality of the rotation of the ultrasound imaging probe 210, including whether the ultrasound imaging probe 210 is rotating towards or away from the ultrasound imaging probe 210 and from the left or the right. The voltage readings are from the passive ultrasound sensor S1. Thus, the changes in voltage from the passive ultrasound sensor S1 and the changes in distance measurements d₀ and d₁ that reflect the rotation ΔΘ are interpreted as a simple reflection of the positioning and directionality of the ultrasound imaging probe 210, which in turn reflects which side of the imaging plane the passive ultrasound sensor S1 is on.

Visualizations E, F, G and H in FIG. 4 are comparative to visualizations A, B, C and D in FIG. 4 and show two additional metrics. A first metric is the parallel distance with respect to the imaging plane from an extremity on the ultrasound imaging probe 210 from which the imaging plane is emitted, to a line perpendicular to the imaging plane that intersects a fixed point on the passive ultrasound sensor S1. The parallel distance is the distance from a fixed point such as the location of the inertial motion unit 212 attached to the ultrasound imaging probe 210 to the emission point from the imaging array of transducers on the inertial motion unit 212, since the assumption is that the emission point is fixed as the ultrasound imaging probe 210 is rolled over a point on the skin without significant translational movement. The second metric is the perpendicular distance to the fixed point on the passive ultrasound sensor S1 from the imaging plane on the line perpendicular to the imaging plane, and this perpendicular distance may be the out-of-plane distance determined at S221 as described herein. That is, the details of visualizations E, F, G and H may apply to all of visualizations A, B, C and D, as the perpendicular distance detailed in visualizations E, G, G and H may be the out-of-plane distance calculated at S221 for each scenario. That is, the horizontal distances explained for visualizations A, B, C and D may be replaced with perpendicular distances explained for visualizations E, F, G and H.

As described above in relation to FIG. 4, four outcomes are possible at all times. A first outcome occurs if the voltage increases with a rotation away from the arbitrary, fixed sensor axis, as the passive ultrasound sensor S1 is on the same side as the rotation. A second outcome occurs if the voltage decreases with a rotation away from the arbitrary, fixed sensor axis, as the passive ultrasound sensor S1 is on the opposite side as the rotation. A third outcome occurs if the voltage increases with a rotation toward the arbitrary, fixed sensor axis, as the passive ultrasound sensor S1 is on the same side as the rotation. The fourth outcome occurs if the voltage decreases with a rotation toward the arbitrary, fixed sensor axis, as the passive ultrasound sensor S1 is on the opposite side as the rotation.

The embodiments above have primarily discussed how out-of-plane directionality and out-of-plane distance are determined for a passive ultrasound sensor S1. However, the position of the passive ultrasound sensor S1 as determined from the out-of-plane directionality and the out-of-plane distance can also be used as an accurate reference marker for three-dimensional volume reconstruction. Three-dimensional volumetric reconstruction around the position of the passive ultrasound sensor S1 can be performed by using the position of the passive ultrasound sensor S1 as a constraint on the out-of-plane translations and rotations measured from the inertial motion unit 212 in FIG. 2A and the inertial motion unit 312 in FIG. 3.

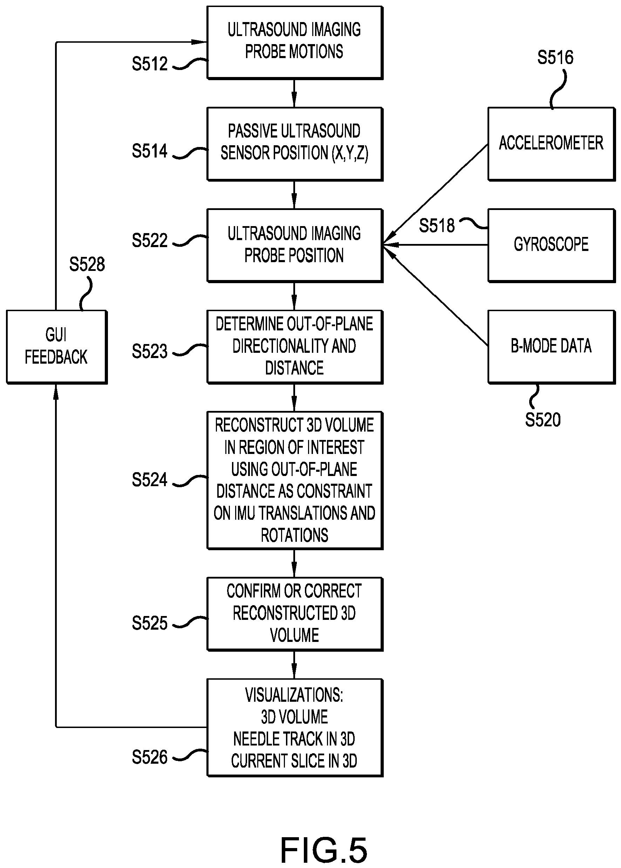

FIG. 5 illustrates another process for relative location determining for passive ultrasound sensors, in accordance with a representative embodiment.

The process of FIG. 5 begins with obtaining probe motions at S512. The probe motions obtained at S512 may be raw data captured in real time by an inertial motion unit 212 as a user moves an ultrasound imaging probe 210 to which the inertial motion unit 212 is attached. The motion of the ultrasound imaging probe 210 may be measured passively or with a probe wobble provided intentionally by the user as described previously at S214 or S220.

At S514, the process of FIG. 5 obtains passive ultrasound sensor positions in three dimensions, i.e., x, y and z. The data obtained at S514 may be based on voltage readings by a passive ultrasound sensor S1, and the positions may be determined by the system (e.g., the controller 250) that determines the positions of the passive ultrasound sensor S1.

At S516, the process of FIG. 5 obtains accelerometer data. Accelerometer data is data obtained by or from an inertial motion unit 212 and is reflective of translations (displacements) of an ultrasound imaging probe 210 along three axes defining a three-dimensional space.

At S518, the process of FIG. 5 obtains gyroscope data. Gyroscope data is data obtained by or from an inertial motion unit 212 and is reflective of rotations (Euler angles) of an ultrasound imaging probe 210 about the three axes defining the three-dimensional space.

At S520, the process of FIG. 5 obtains B-mode data. B-mode stands for "brightness mode" and refers to the use of an ultrasound imaging probe 210 to emit an ultrasound imaging beam in an imaging plane to obtain a two-dimensional ultrasound image. B-mode data may include a sequence and/or pattern of the ultrasound imaging beams, emission timings of the ultrasound imaging beams, and the two-dimensional ultrasound images that result from the emission of the ultrasound imaging beams. The B-mode data may be processed to obtain image-based or speckle-based features that are useful in determining ultrasound probe motion.

At S522, the process of FIG. 5 determines an ultrasound imaging probe position. The position of an ultrasound imaging probe 210 may be determined at S522 by an inertial motion unit 212 or using data from an inertial motion unit 212. For example, the position of the ultrasound imaging probe 210 may be determined using the accelerometer data from S516 and/or the gyroscope data from S518. Additionally or alternatively, B-mode data from S520 may be used to determine the position of the ultrasound imaging probe 210.

At S524, the process of FIG. 5 generates a three-dimensional volume in the region of interest. As described herein, the three-dimensional volume may be a three-dimensional volumetric reconstruction around the position of the passive ultrasound sensor S1, and the out-of-plane distance between the imaging plane of/from the ultrasound imaging probe 210 and the position of the passive ultrasound sensor S1 may be used as a constraint on the out-of-plane translations and rotations measured from the inertial motion unit 212.

At S526, the process of FIG. 5 may be used to generate visualizations of the three-dimensional volume, a track of the interventional medical device in the three-dimensional volume, and a current slice in the three-dimensional volume. The processes of determining out-of-plane directionality and out-of-plane distance as described above may be extended to provide local three-dimensional volume reconstructions around the position of the passive ultrasound sensor S1. Here, the out-of-plane distance between the ultrasound imaging plane and the position of the passive ultrasound sensor S1 is or may be used as a constraint on the out-of-plane translational component of the pose transformation provided by the inertial motion unit 212. The workflow may start with a user providing a request or instruction to generate or otherwise obtain a three-dimensional volume. The user then creates a volumetric sweep starting from one side of the passive ultrasound sensor S1 and ending at the other. Ideally, the sweep is approximately symmetric around the position of the passive ultrasound sensor S1. Next, the translational and rotational components of the movement of the ultrasound imaging probe 210 are estimated at each time point using the inertial motion unit 212 and/or the controller 250, as described above. Individual frames are then reconstructed to form the three-dimensional volume at S526. Errors are expected in the volume estimation due to the imprecision of the readings of the inertial motion unit 212 as well as position drift.

Based on the processes at steps S514, S516 and S518, the out-of-plane directionality and distance are or may be used then as a constraint and consistency check applied to confirm the reconstructed three-dimensional volume or detect and correct for errors in the three-dimensional reconstruction at S525. The checks include detecting inconsistencies in the out-of-plane distance/direction between individual frames in the three-dimensional volume by applying rules described herein. As a result, inconsistent frames may then be removed from the three-dimensional reconstruction. Alternatively or additionally, the pose of the frames may be adjusted by bringing the frame closer to the "expected" pose according to the readings of the location system for the passive ultrasound sensor S1. Another check may be performed by ensuring that the frames deemed to be in-plane according to the three-dimensional reconstruction correspond to frames having maximum voltages from the passive ultrasound sensor S1. That is, plane crossings as described herein can be determined based on the location system for the passive ultrasound sensor S1 and these crossings will be reflected in the three-dimensional reconstruction estimated based on pose estimations derived from the inertial motion unit 212. A further check is based on the assumption that the out-of-plane profile for the passive ultrasound sensor S1 is symmetric about the maximum voltage. As a result, the out-of-plane frame-to-frame spacing of the three-dimensional volume may also be symmetric relative to the voltage of the passive ultrasound sensor S1. That is, a 1 millimeter frame-to-frame distance should correspond to the same magnitude drop in voltage on one side of the in-plane axis as on the other side. Out-of-plane rotation characterized by roll and yaw should be similarly consistent with changes in the response of the passive ultrasound sensor S1.

Since the location system for the passive ultrasound sensor S1 cannot differentiate two types of motion, rotational motions may be difficult to separate from translational motions. Here, if more than one passive ultrasound sensor S1 is within the ultrasound field of view, accuracy may be further improved compared to the presence of only a single passive ultrasound sensor S1. Manual calibration can be used as a further check for accuracy. For example, a user may be enabled or even prompted to calibrate in a patient-specific manner the voltage measurements of the passive ultrasound sensor voltage S1 to the measurements by the inertial motion unit 212 and/or image-based measurements. Because accuracy of the inertial motion unit 212 is proportional to the frame-to-frame acceleration/deceleration, the user may be prompted to perform a calibration step involving a rotation or translation at high speed. The more accurate measurements of the inertial motion unit 212 may then be related to the voltage drop-off of the passive ultrasound sensor S1, creating a calibration curve for the motions during the interventional procedure.

At S526, the visualizations of the reconstructed three-dimensional volume may include the three-dimensional volume, a track of the interventional medical device 205 in the three-dimensional volume, and a current slice in the three-dimensional volume. For example, the interventional medical device 205 may be a needle, so the track may be a needle track in the three-dimensional volume of the region of interest. The current slice may be highlighted in the three-dimensional volume by selectively adjusting the brightness or color of the current slice, or by adding or updating a border for the current slice in the context of the three-dimensional volume

At S528, the process of FIG. 5 obtains GUI feedback, and then returns to the beginning at S512 to obtain probe motions. The GUI feedback may be input from a user via a graphical user interface. The input from a user may be confirmation of the visualizations generated at S526, or a request to retry or update the process of FIG. 5.

As described above in the context of FIG. 5, a three-dimensional volume may be reconstructed using constraints such as the location of a passive ultrasound sensor S1. The location determination itself may also be confirmed or updated based on accuracy checks of any of a variety of types including speckle-based decorrelation. A process for speckle decorrelation for relative location determining for passive ultrasound sensors may be used consistent with the present disclosure, in accordance with a representative embodiment. For estimating the out-of-plane motion, measuring the decorrelation of speckle features in the ultrasound image can be used as an approximation of out-of-plane translational movement. The overlap of the imaging beam widths during out-of-plane movements results in correlation in the speckle between adjacent frames. The amount of correlation, which may be quantified by analyzing patches in each frame, can be used to predict the frame-to-frame distance. The limitation of these approaches has traditionally been the difficulty in computing accurate translational frame distances in the presence of unknown rotation. However, since the rotation is determined as described herein using the inertial motion unit 212 or another mechanism, speckle decorrelation methods can be used and made useful since the accuracy of such speckle decorrelation is significantly improved. A speckle decorrelation technique may therefore be used for estimating out-of-plane translational motion. The additional use of image-based tracking techniques, including intensity-based tracking and speckle-based tracking, is or may be used to further refine the estimates of pose of the ultrasound imaging probe, such as in S525 in FIG. 5. Specifically, an overlap of beam widths during out-of-plane translations may be identified at a first time. The overlap may be used to generate (e.g., identify, calculate, determine) the correlation between adjacent frames ft..ft+1 at a second time. The degree of correlation ρ is used to predict the out-of-plane distance d between frames at a third time.

Incorporating image-based speckle decorrelation tracking for estimating out-of-plane motion may be a form of refinement using image-based information. The refinement further confirms or corrects the out-of-plane pose estimates of the ultrasound imaging probe 210, as well as the three-dimensional volume reconstruction. For example, the decorrelation of speckle features in the ultrasound image can provide an approximation of out-of-plane translation. Here, the overlap of the imaging beam widths during out-of-plane movements results in correlation in the speckle between adjacent frames. The amount of correlation, which may be quantified by analyzing patches in each frame, can be used to predict the frame-to-frame distance.

The speckle decorrelation technique can be incorporated into the previously described workflow of estimation using a passive ultrasound sensor S1 and pose estimation and reconstruction based on readings of an inertial motion unit 212. Specifically, since the gyroscope of the inertial motion unit 212 is able to accurately measure rotations, and the passive ultrasound sensor S1 provides additional constraints, the translational component of the motion is more separable. The magnitude of the translation may then be estimated based on speckle decorrelation. With the response of the passive ultrasound sensor S1 in the field-of-view of the ultrasound images acquired during the three-dimensional volume sweep, out-of-plane speckle decorrelation of the response of the passive ultrasound sensor S1 may be measured as well and correlated to out-of-plane distance. Finally, whereas speckle decorrelation estimates out-of-plane translation, intensity-based image tracking methods can be used to estimate in-plane translation. These techniques can similarly be included with the processes described herein to improve accuracy.

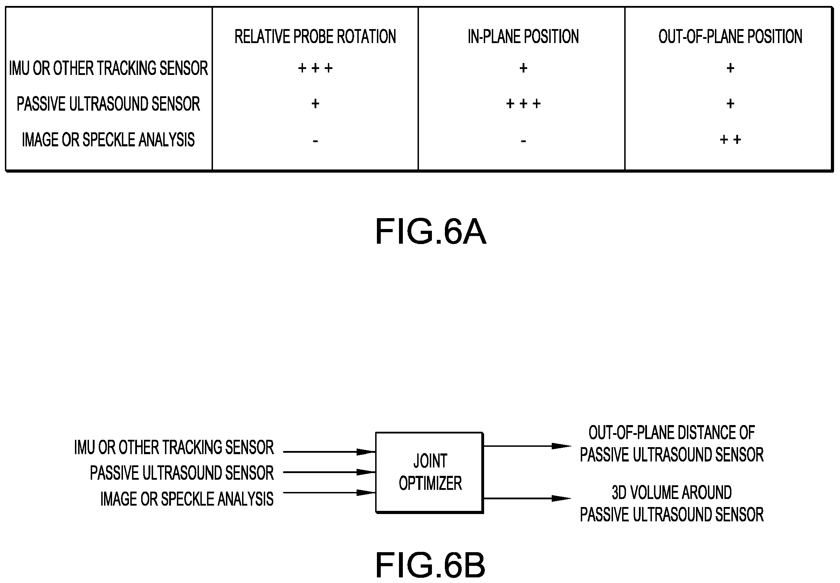

FIG. 6A illustrates input data for obtaining a three-dimensional probe pose in relative location determining for passive ultrasound sensors, in accordance with a representative embodiment.

In FIG. 6A and FIG. 6B, passive ultrasound sensor tracking, inertial motion unit tracking and speckle-based tracking are provided for improved three-dimensional out-of-plane estimation and reconstruction. In FIG. 6A, three measurements are obtained in order to identify or otherwise obtain the accurate three-dimensional pose of the interventional medical device 205. The three measurements are rotation of the ultrasound imaging probe 210 (i.e., from the inertial motion unit tracking), in-plane position of the ultrasound imaging probe 210, and out-of-plane position of the ultrasound imaging probe 210. Each of the three measurements may be obtained from a different information source.

FIG. 6B illustrates inputs and outputs for joint optimization for obtaining a three-dimensional probe pose in relative location determining for passive ultrasound sensors, in accordance with a representative embodiment.

In FIG. 6B, out-of-plane distance and pose of the ultrasound imaging probe 210 may be determined more accurately by combining the different information sources, for example using a joint optimization as described herein. The individual image information from the ultrasound imaging frame poses may then be reconstructed to form a more accurate three-dimensional volume compared to an approach that relies only on one type of information source. Specifically, the position of the passive ultrasound sensor S1 is used to serve as a high-accuracy reference point that constrains the optimization from producing incorrect solutions. The use of passive ultrasound sensor tracking to constrain estimates of inertial motion unit pose for reconstructing three-dimensional volumes around the device tip was shown and explained above.

In FIG. 6B, constraints are or may be applied within a single framework by using an optimization scheme. Here, a penalty may be provided for each violated constraint. An optimization algorithm used as/for the optimization scheme attempts to determine a full set of transducer pose parameters that violates the fewest constraints while remaining in close agreement with the original measurements of the inertial motion unit 212. In an embodiment, the relative importance of the measurements from the inertial motion unit 212 and from the passive ultrasound sensor S1 may be a user-defined weighting factor, and these can be used to govern how the corrections are made. That is, a user-defined weighting factor for sensor measurements may be used to govern whether individual frames are corrected to match closer to the result of the passive ultrasound sensor S1 or the result of the inertial motion unit 212. The weighting factor may be introduced in the optimization as a constant parameter applied to each constraint, thus dictating the magnitude of the penalty if that constraint were violated. In another embodiment, the relative weights may be learned in a calibration step during manufacturing, for example by attaching a high-accuracy "ground-truth" external tracking sensor such as an electromagnetic or optical sensor, so that results of the passive ultrasound sensor S1 and/or the inertial motion unit 212 can always be compared to the ground truth to weight based on relative accuracy.

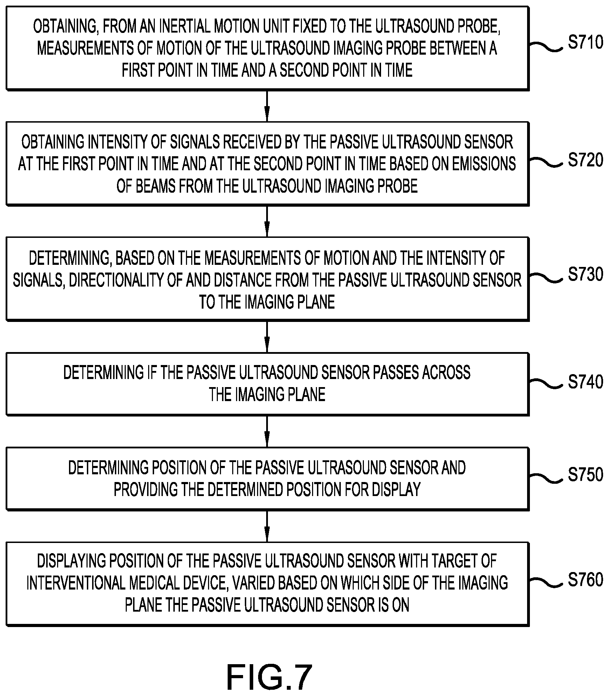

FIG. 7 illustrates another process for relative location determining for passive ultrasound sensors, in accordance with a representative embodiment.

The process in FIG. 7 starts at S710 by obtaining, from an inertial motion unit fixed to the ultrasound imaging probe, measurements of motion of the ultrasound imaging probe between a first point in time and a second point in time. The measurements obtained at S710 may be obtained by a controller 250 from an inertial motion unit 212 fixed to an ultrasound imaging probe 210.

At S720, the process of FIG. 7 proceeds by obtaining intensity of signals received by the passive ultrasound sensor at the first point in time and at the second point in time based on emissions of beams from the ultrasound imaging probe. The intensity obtained at S720 may be obtained by a controller 250 from a passive ultrasound sensor S1.

At S730, the process of FIG. 7 next includes determining, based on the measurements of motion and the intensity of signals, directionality of and distance from the passive ultrasound sensor to the imaging plane. Out-of-plane directionality and out-of-plane distance may each be determined by a controller 250, as separately described herein. At S740 the process of FIG. 7 includes determining if the passive ultrasound sensor passes across the imaging plane. The determination at S750 may be performed by a controller 250 and may involve determining when peak voltage readings occur during operations involving ultrasound imaging during an interventional procedure.

At S750, the process of FIG. 7 includes determining the position of the passive ultrasound sensor and providing the determined position for display. Determining the position of the passive ultrasound sensor S1 at S750 may be based, in part, on the directionality and out-of-plane distance determinations at S730.

At S760, the process of FIG. 7 includes displaying the position of the passive ultrasound sensor with the target of the interventional medical device, varied based on which side of the imaging plane the passive ultrasound sensor is on. The varying at S760 may be by color, brightness, icon and so on.

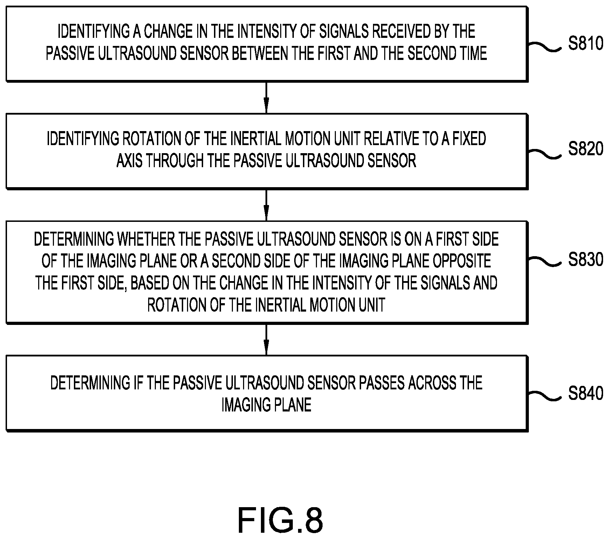

FIG. 8 illustrates another process for relative location determining for passive ultrasound sensors, in accordance with a representative embodiment.

The process of FIG. 8 begins at S810 by identifying a change in the intensity of signals received by the passive ultrasound sensor between the first time and the second time.

Next, the process of FIG. 8 proceeds to S820 by identifying rotation of the inertial motion unit relative to a fixed axis through the passive ultrasound sensor.

At S830, the process of FIG. 8 includes determining whether the passive ultrasound sensor is on a first side of the imaging plane or a second side of the imaging plane opposite the first side, based on the change in the intensity of the signals and rotation of the inertial motion unit.

At S840, the process of FIG. 8 concludes with determining if the passive ultrasound sensor passes across the imaging plane.

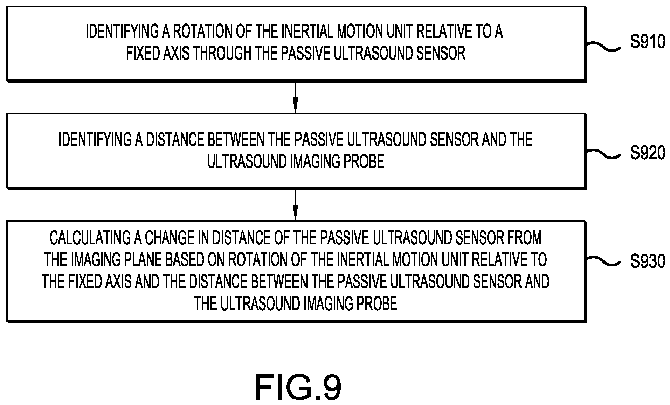

FIG. 9 illustrates another process for relative location determining for passive ultrasound sensors, in accordance with a representative embodiment.

The process of FIG. 9 begins at S910 by identifying a rotation of the inertial motion unit relative to a fixed axis through the passive ultrasound sensor.

At S920, the process of FIG. 9 includes identifying a distance between the passive ultrasound sensor and the ultrasound imaging probe.

At S930, the process of FIG. 9 ends with calculating a change in the distance of the passive ultrasound sensor from the imaging plane based on the rotation of the inertial motion unit relative to the fixed axis and the distance between the passive ultrasound sensor and the ultrasound imaging probe.

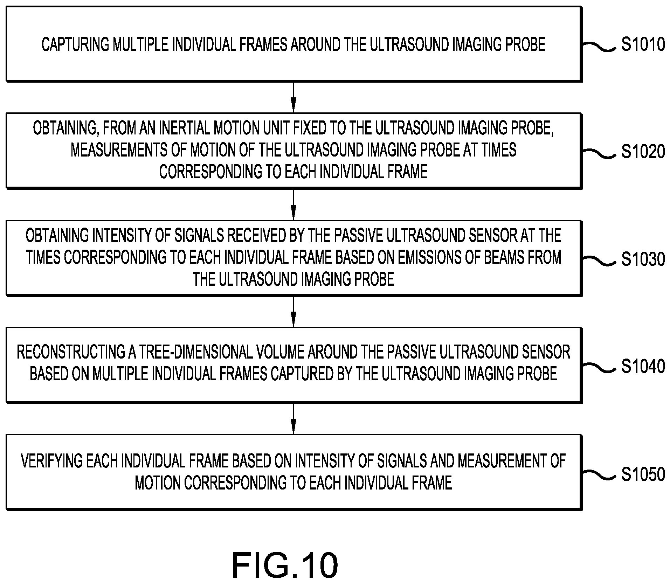

FIG. 10 illustrates another process for relative location determining for passive ultrasound sensors, in accordance with a representative embodiment.

The process of FIG. 10 starts at S1010 by capturing multiple individual frames around the ultrasound imaging probe.

Next, at S1020 the process of FIG. 10 includes obtaining, from an inertial motion unit fixed to the ultrasound imaging probe, measurements of motion of the ultrasound imaging probe corresponding to each individual frame.

At S1030 the process of FIG. 10 includes obtaining intensity of signals received by the passive ultrasound sensor at the times corresponding to each individual frame based on emissions of beams from the ultrasound imaging probe.

At S1040, the process of FIG. 10 next includes reconstructing the three-dimensional volume around the passive ultrasound sensor based on multiple individual frames captured by the ultrasound imaging probe.

At S1050, the process of FIG. 10 concludes with verifying each individual frame based on the intensity of signals and measurements of motion corresponding to each individual frame.

Accordingly, relative location determining for passive ultrasound sensors enables significant reduction in the error that typically builds up over time with inertial sensing methods, for example due to position drift, so long as the passive ultrasound sensor S1 remains still. Accuracy can be improved with additional methods described herein in which image-based information is incorporated, either as an alternative to IMU tracking or in addition to IMU. Although relative location determining for passive ultrasound sensors has been described with reference to several exemplary embodiments, it is understood that the words that have been used are words of description and illustration, rather than words of limitation. Changes may be made within the purview of the appended claims, as presently stated and as amended, without departing from the scope and spirit of relative location determining for passive ultrasound sensors in its aspects. Although relative location determining for passive ultrasound sensors has been described with reference to particular means, materials and embodiments, relative location determining for passive ultrasound sensors is not intended to be limited to the particulars disclosed; rather relative location determining for passive ultrasound sensors extends to all functionally equivalent structures, methods, and uses such as are within the scope of the appended claims.

For example, relative location determining for passive ultrasound sensors may be applied to many and perhaps all tracked interventional procedures. Identifying (e.g., calculating, determining, estimating) the out-of-plane distance between a device tip and tissue target may be important in many different types of interventional procedures, and relative location determining for passive ultrasound sensors may allow such functionality with relatively low development overhead. The distance identification can also be used to help provide better three-dimensional context, and learning for new users, which in turn may increase customer confidence during procedures and add value to systems and devices that are equipped with tracking such as with passive ultrasound sensors.

The teachings of relative location determining for passive ultrasound sensors can be used to improve, for example, vascular access, insofar as knowing the out-of-plane distance between the tip of the interventional medical device 105 and the vessel target may be important to insertion accuracy. Similarly, the teachings of relative location determining for passive ultrasound sensors can be used to determine when an inserted guidewire as the interventional medical device 105 crosses the center of an intravascular lesion (intraluminal crossing) or when the guidewire has redirected toward the vessel wall (subintimal crossing), so as to aid in avoiding vessel wall perforation.

The following Examples are provided:
Example 1. A controller (250) for identifying out-of-plane motion of a passive ultrasound sensor (S1) relative to an imaging plane from an ultrasound imaging probe (210), comprising:
   a memory (391) that stores instructions, and
   a processor (392) that executes the instructions, wherein, when executed by the processor (392), the instructions cause a system that includes the controller (250) to implement a process that includes:
      obtaining (S710), from a position and orientation sensor (212) fixed to the ultrasound imaging probe (210), measurements of motion of the ultrasound imaging probe (210) between a first point in time and a second point in time;
      obtaining (S720) intensity of signals received by the passive ultrasound sensor (S1) at the first point in time and at the second point in time based on emissions of beams from the ultrasound imaging probe (210), and
      determining (S730), based on the measurements of motion and the intensity of signals, directionality of and distance from the passive ultrasound sensor (S1) to the imaging plane.
Example 2. The controller (250) of Example 1, wherein the determining further comprises:
   identifying (S810) a change in the intensity of signals received by the passive ultrasound sensor (S1) between the first point in time and the second point in time;
   identifying (S820) rotation of the position and orientation sensor (212) relative to a fixed axis through the passive ultrasound sensor, and
   determining (S830) whether the passive ultrasound sensor (S1) is on a first side of the imaging plane or a second side of the imaging plane opposite the first side, based on the change in the intensity of signals and rotation of the position and orientation sensor (212).
Example 3. The controller (250) of Example 1, wherein the determining further comprises:
   identifying (S910) rotation of the position and orientation sensor (212) relative to a fixed axis through the passive ultrasound sensor;
   identifying (S920) a distance between the passive ultrasound sensor (S1) and the ultrasound imaging probe (210); and
   calculating (S930) a change in distance of the passive ultrasound sensor (S1) from the imaging plane based on rotation of the position and orientation sensor (212) relative to the fixed axis and the distance between the passive ultrasound sensor (S1) and the ultrasound imaging probe (210).
Example 4. The controller (250) of Example 3, wherein the distance of the passive ultrasound sensor (S1) from the imaging plane is determined between the imaging plane and a fixed point on the fixed axis of the passive ultrasound sensor (S1) along a line perpendicular to the imaging plane at the first point in time and the second point in time.
Example 5. The controller (250) of Example 1,
   wherein the passive ultrasound sensor (S1) is fixed to an interventional medical device (205), and
   the process implemented by the system further comprises providing (S760) a position of the passive ultrasound sensor (S1) for display together with a target of the interventional medical device (205).
Example 6. The controller (250) of Example 1, wherein the position and orientation sensor (212) comprises an accelerometer that measures three-dimensional translations of the ultrasound imaging probe (210) and a gyroscope that measures three-dimensional rotations of the ultrasound imaging probe (210).
Example 7. The controller (250) of Example 1, wherein the process implemented by the system further comprises:
   determining (S830) whether the passive ultrasound sensor (S1) is on a first side of the imaging plane or a second side of the imaging plane opposite the first side, based on a change in the intensity of signals and the measurements of motion of the ultrasound imaging probe (210), and
   determining (S840) when the passive ultrasound sensor (S1) passes across the imaging plane from the first side to the second side.
Example 8. The controller (250) of Example 7, wherein the process implemented by the controller (250) further comprises:
   controlling (S760) a displayed representation of the passive ultrasound sensor (S1) to vary based on whether the passive ultrasound sensor (S1) is on the first side of the imaging plane or the second side of the imaging plane.
Example 9. The controller (250) of Example 1, wherein the process implemented by the controller (250) further comprises:
   reconstructing (SI040) a three-dimensional volume around the passive ultrasound sensor (S1) based on a plurality of individual frames captured by the ultrasound imaging probe (210); and
   verifying (S1050) each of the plurality of individual frames based on the intensity of signals and the measurements of motion corresponding to each of the plurality of individual frames.
Example 10. A tangible non-transitory computer readable storage medium (391) that stores a computer program, the computer program, when executed by a processor (392), causing a system that includes the tangible non-transitory computer readable storage medium to perform a process for identifying out-of-plane motion of a passive ultrasound sensor (S1) relative to an imaging plane from an ultrasound imaging probe (310), the process performed when the processor (392) executes the computer program from the tangible non-transitory computer readable storage medium comprising:
   obtaining (S710), from a position and orientation sensor (312) fixed to the ultrasound imaging probe (310), measurements of motion of the ultrasound imaging probe (310) between a first point in time and a second point in time;
   obtaining (S720) intensity of signals received by the passive ultrasound sensor (S1) at the first point in time and at the second point in time based on emissions of beams from the ultrasound imaging probe (310), and
   determining (S730), based on the measurements of motion and the intensity of signals, directionality of and distance from the passive ultrasound sensor (S1) to the imaging plane.
Example 11. The tangible non-transitory computer readable storage medium (391) of Example 10, wherein the determining further comprises:
   identifying (S810) a change in the intensity of signals received by the passive ultrasound sensor (S1) between the first point in time and the second point in time;
   identifying (S820) rotation of the position and orientation sensor (312) relative to a fixed axis through the passive ultrasound sensor, and
   determining (S830) whether the passive ultrasound sensor (S1) is on a first side of the imaging plane or a second side of the imaging plane opposite the first side, based on the change in the intensity of signals and rotation of the position and orientation sensor (312).
Example 12. The tangible non-transitory computer readable storage medium (391) of Example 10, wherein the determining further comprises:
   identifying (S910) rotation of the position and orientation sensor (312) relative to a fixed axis through the passive ultrasound sensor;
   identifying (S920) a distance between the passive ultrasound sensor (S1) and the ultrasound imaging probe (310); and
   calculating (S930) a change in distance of the passive ultrasound sensor (S1) from the imaging plane based on rotation of the position and orientation sensor (312) relative to the fixed axis and the distance between the passive ultrasound sensor (S1) and the ultrasound imaging probe (310).
Example 13. The tangible non-transitory computer readable storage medium (391) of Example 12, wherein the distance of the passive ultrasound sensor (S1) from the imaging plane is determined from a fixed point on the fixed axis through the passive ultrasound sensor (S1) to an intersection between the imaging plane and a line perpendicular to the fixed axis from the fixed point at the first point in time and the second point in time.
Example 14. The tangible non-transitory computer readable storage medium (391) of Example 10,
   wherein the passive ultrasound sensor (S1) is fixed to an interventional medical device (301), and
   the process implemented by the system further comprises providing (S760) a position of the passive ultrasound sensor (S1) for display together with a target of the interventional medical device (301).
Example 15. The tangible non-transitory computer readable storage medium (391) of Example 10, wherein the position and orientation sensor (312) comprises an accelerometer that measures three-dimensional translations of the ultrasound imaging probe (310) and a gyroscope that measures three-dimensional rotations of the ultrasound imaging probe (310).
Example 16. The tangible non-transitory computer readable storage medium (391) of Example 10, wherein the process implemented by the system further comprises:
   determining (S830) whether the passive ultrasound sensor (S1) is on a first side of the imaging plane or a second side of the imaging plane opposite the first side, based on a change in the intensity of signals and the measurements of motion of the ultrasound imaging probe (310); and
   determining (S840) when the passive ultrasound sensor (S1) passes across the imaging plane from the first side to the second side.
Example 17. The tangible non-transitory computer readable storage medium (391) of Example 16, wherein the process implemented by the system further comprises:
   controlling (S760) a displayed representation of the passive ultrasound sensor (S1) to vary based on whether the passive ultrasound sensor (S1) is on the first side of the imaging plane or the second side of the imaging plane.
Example 18. A system (300) for identifying out-of-plane motion of a passive ultrasound sensor (S1) relative to an imaging plane from an ultrasound imaging probe (310), comprising:
   an ultrasound imaging probe (310) that emits beams during a medical intervention;
   a position and orientation sensor (312) fixed to the ultrasound imaging probe (310);
   a passive ultrasound sensor (S1) fixed to an interventional medical device (301) during the medical intervention; and
   a controller (250) comprising a memory (391) that stores instructions and a processor (392) that executes the instructions, wherein, when executed by the processor (392), the instructions cause the system (300) to implement a process that includes:
      obtaining (S710), from the position and orientation sensor (312), measurements of motion of the ultrasound imaging probe (310) between a first point in time and a second point in time;
      obtaining (S720) intensity of signals received by the passive ultrasound sensor (S1) at the first point in time and at the second point in time based on emissions of beams from the ultrasound imaging probe (310);
      determining (S730), based on the measurements of motion and the intensity of signals, directionality of and distance from the passive ultrasound sensor (S1) to the imaging plane.
Example 19. The system of Example 18, wherein the determining further comprises:
   identifying (S810) a change in the intensity of signals received by the passive ultrasound sensor (S1) between the first point in time and the second point in time;
   identifying (S820) rotation of the position and orientation sensor (312) relative to a fixed axis through the passive ultrasound sensor, and
   determining (S830) whether the passive ultrasound sensor (S1) is on a first side of the imaging plane or a second side of the imaging plane opposite the first side, based on the change in the intensity of signals and rotation of the position and orientation sensor (312).
Example 20. The system of Example 18, wherein the determining further comprises:
   identifying (S910) rotation of the position and orientation sensor (312) relative to a fixed axis through the passive ultrasound sensor;
   identifying (S920) a distance between the passive ultrasound sensor (S1) and the ultrasound imaging probe (310); and
   calculating (S930) a change in distance of the passive ultrasound sensor (S1) from the imaging plane based on rotation of the position and orientation sensor (312) relative to the fixed axis and the distance between the passive ultrasound sensor (S1) and the ultrasound imaging probe (310).

The illustrations of the embodiments described herein are intended to provide a general understanding of the structure of the various embodiments. The illustrations are not intended to serve as a complete description of all of the elements and features of the disclosure described herein. Many other embodiments may be apparent to those of skill in the art upon reviewing the disclosure. Other embodiments may be utilized and derived from the disclosure, such that structural and logical substitutions and changes may be made without departing from the scope of the disclosure. Additionally, the illustrations are merely representational and may not be drawn to scale. Certain proportions within the illustrations may be exaggerated, while other proportions may be minimized. Accordingly, the disclosure and the figures are to be regarded as illustrative rather than restrictive.

One or more embodiments of the disclosure may be referred to herein, individually and/or collectively, by the term "invention" merely for convenience and without intending to voluntarily limit the scope of this application to any particular invention or inventive concept. Moreover, although specific embodiments have been illustrated and described herein, it should be appreciated that any subsequent arrangement designed to achieve the same or similar purpose may be substituted for the specific embodiments shown. This disclosure is intended to cover any and all subsequent adaptations or variations of various embodiments. Combinations of the above embodiments, and other embodiments not specifically described herein, will be apparent to those of skill in the art upon reviewing the description.

The Abstract of the Disclosure is provided to comply with 37 C.F.R. § 1.72(b) and is submitted with the understanding that it will not be used to interpret or limit the scope or meaning of the claims. In addition, in the foregoing Detailed Description, various features may be grouped together or described in a single embodiment for the purpose of streamlining the disclosure. This disclosure is not to be interpreted as reflecting an intention that the claimed embodiments require more features than are expressly recited in each claim. Rather, as the following claims reflect, inventive subject matter may be directed to less than all of the features of any of the disclosed embodiments. Thus, the following claims are incorporated into the Detailed Description, with each claim standing on its own as defining separately claimed subject matter.

The preceding description of the disclosed embodiments is provided to enable any person skilled in the art to practice the concepts described in the present disclosure. As such, the above disclosed subject matter is to be considered illustrative, and not restrictive, and the appended claims are intended to cover all such modifications, enhancements, and other embodiments which fall within the true spirit and scope of the present disclosure. Thus, to the maximum extent allowed by law, the scope of the present disclosure is to be determined by the broadest permissible interpretation of the following claims and their equivalents and shall not be restricted or limited by the foregoing detailed description.

## Claims

1. A controller (250) for identifying out-of-plane motion of a passive ultrasound sensor (S1) relative to an imaging plane from an ultrasound imaging probe (210), comprising:
a memory (391) that stores instructions, and
a processor (392) that executes the instructions, wherein, when executed by the processor (392), the instructions cause a system that includes the controller (250) to implement a process that includes:
obtaining (S710), from a position and orientation sensor (212) fixed to the ultrasound imaging probe (210), measurements of motion of the ultrasound imaging probe (210) between a first point in time and a second point in time;
obtaining (S720) intensity of signals received by the passive ultrasound sensor (S1) at the first point in time and at the second point in time based on emissions of beams from the ultrasound imaging probe (210), and
determining (S730), based on the measurements of motion and the intensity of signals, directionality of and distance from the passive ultrasound sensor (S1) to the imaging plane.

2. The controller (250) of claim 1, wherein the determining further comprises:
identifying (S810) a change in the intensity of signals received by the passive ultrasound sensor (S1) between the first point in time and the second point in time;
identifying (S820) rotation of the position and orientation sensor (212) relative to a fixed axis through the passive ultrasound sensor, and
determining (S830) whether the passive ultrasound sensor (S1) is on a first side of the imaging plane or a second side of the imaging plane opposite the first side, based on the change in the intensity of signals and rotation of the position and orientation sensor (212).

3. The controller (250) of claim 1, wherein the determining further comprises:
identifying (S910) rotation of the position and orientation sensor (212) relative to a fixed axis through the passive ultrasound sensor;
identifying (S920) a distance between the passive ultrasound sensor (S1) and the ultrasound imaging probe (210); and
calculating (S930) a change in distance of the passive ultrasound sensor (S1) from the imaging plane based on rotation of the position and orientation sensor (212) relative to the fixed axis and the distance between the passive ultrasound sensor (S1) and the ultrasound imaging probe (210).

4. The controller (250) of claim 3, wherein the distance of the passive ultrasound sensor (S1) from the imaging plane is determined between the imaging plane and a fixed point on the fixed axis of the passive ultrasound sensor (S1) along a line perpendicular to the imaging plane at the first point in time and the second point in time.

5. The controller (250) of claim 1,
wherein the passive ultrasound sensor (S1) is fixed to an interventional medical device (205), and
the process implemented by the system further comprises providing (S760) a position of the passive ultrasound sensor (S1) for display together with a target of the interventional medical device (205).

6. The controller (250) of claim 1, wherein the position and orientation sensor (212) comprises an accelerometer that measures three-dimensional translations of the ultrasound imaging probe (210) and a gyroscope that measures three-dimensional rotations of the ultrasound imaging probe (210).

7. The controller (250) of claim 1, wherein the process implemented by the system further comprises:
determining (S830) whether the passive ultrasound sensor (S1) is on a first side of the imaging plane or a second side of the imaging plane opposite the first side, based on a change in the intensity of signals and the measurements of motion of the ultrasound imaging probe (210), and
determining (S840) when the passive ultrasound sensor (S1) passes across the imaging plane from the first side to the second side.

8. The controller (250) of claim 7, wherein the process implemented by the controller (250) further comprises:
controlling (S760) a displayed representation of the passive ultrasound sensor (S1) to vary based on whether the passive ultrasound sensor (S1) is on the first side of the imaging plane or the second side of the imaging plane.

9. The controller (250) of claim 1, wherein the process implemented by the controller (250) further comprises:
reconstructing (SI040) a three-dimensional volume around the passive ultrasound sensor (S1) based on a plurality of individual frames captured by the ultrasound imaging probe (210); and
verifying (S1050) each of the plurality of individual frames based on the intensity of signals and the measurements of motion corresponding to each of the plurality of individual frames.

10. A tangible non-transitory computer readable storage medium (391) that stores a computer program, the computer program, when executed by a processor (392), causing a system that includes the tangible non-transitory computer readable storage medium to perform a process for identifying out-of-plane motion of a passive ultrasound sensor (S1) relative to an imaging plane from an ultrasound imaging probe (310), the process performed when the processor (392) executes the computer program from the tangible non-transitory computer readable storage medium comprising:
obtaining (S710), from a position and orientation sensor (312) fixed to the ultrasound imaging probe (310), measurements of motion of the ultrasound imaging probe (310) between a first point in time and a second point in time;
obtaining (S720) intensity of signals received by the passive ultrasound sensor (S1) at the first point in time and at the second point in time based on emissions of beams from the ultrasound imaging probe (310), and
determining (S730), based on the measurements of motion and the intensity of signals, directionality of and distance from the passive ultrasound sensor (S1) to the imaging plane.

11. The tangible non-transitory computer readable storage medium (391) of claim 10, wherein the determining further comprises:
identifying (S810) a change in the intensity of signals received by the passive ultrasound sensor (S1) between the first point in time and the second point in time;
identifying (S820) rotation of the position and orientation sensor (312) relative to a fixed axis through the passive ultrasound sensor, and
determining (S830) whether the passive ultrasound sensor (S1) is on a first side of the imaging plane or a second side of the imaging plane opposite the first side, based on the change in the intensity of signals and rotation of the position and orientation sensor (312).

12. The tangible non-transitory computer readable storage medium (391) of claim 10, wherein the determining further comprises:
identifying (S910) rotation of the position and orientation sensor (312) relative to a fixed axis through the passive ultrasound sensor;
identifying (S920) a distance between the passive ultrasound sensor (S1) and the ultrasound imaging probe (310); and
calculating (S930) a change in distance of the passive ultrasound sensor (S1) from the imaging plane based on rotation of the position and orientation sensor (312) relative to the fixed axis and the distance between the passive ultrasound sensor (S1) and the ultrasound imaging probe (310).

13. A system (300) for identifying out-of-plane motion of a passive ultrasound sensor (S1) relative to an imaging plane from an ultrasound imaging probe (310), comprising:
an ultrasound imaging probe (310) that emits beams during a medical intervention;
a position and orientation sensor (312) fixed to the ultrasound imaging probe (310);
a passive ultrasound sensor (S1) fixed to an interventional medical device (301) during the medical intervention; and
a controller (250) comprising a memory (391) that stores instructions and a processor (392) that executes the instructions, wherein, when executed by the processor (392), the instructions cause the system (300) to implement a process that includes:
obtaining (S710), from the position and orientation sensor (312), measurements of motion of the ultrasound imaging probe (310) between a first point in time and a second point in time;
obtaining (S720) intensity of signals received by the passive ultrasound sensor (S1) at the first point in time and at the second point in time based on emissions of beams from the ultrasound imaging probe (310);
determining (S730), based on the measurements of motion and the intensity of signals, directionality of and distance from the passive ultrasound sensor (S1) to the imaging plane.

14. The system of claim 13, wherein the determining further comprises:
identifying (S810) a change in the intensity of signals received by the passive ultrasound sensor (S1) between the first point in time and the second point in time;
identifying (S820) rotation of the position and orientation sensor (312) relative to a fixed axis through the passive ultrasound sensor, and
determining (S830) whether the passive ultrasound sensor (S1) is on a first side of the imaging plane or a second side of the imaging plane opposite the first side, based on the change in the intensity of signals and rotation of the position and orientation sensor (312).

15. The system of claim 13, wherein the determining further comprises:
identifying (S910) rotation of the position and orientation sensor (312) relative to a fixed axis through the passive ultrasound sensor;
identifying (S920) a distance between the passive ultrasound sensor (S1) and the ultrasound imaging probe (310); and
calculating (S930) a change in distance of the passive ultrasound sensor (S1) from the imaging plane based on rotation of the position and orientation sensor (312) relative to the fixed axis and the distance between the passive ultrasound sensor (S1) and the ultrasound imaging probe (310).
